# EUROPÄISCHE PATENTSCHRIFT

(11) **EP 3 013 343 B1**
(45) Veröffentlichungstag und Bekanntmachung des Hinweises auf die Patenterteilung: **08.08.2018**
(21) Anmeldenummer: 14733198.7
(22) Anmeldetag: 27.06.2014
(51) Int. Cl.: A61K 31/439, A61K 31/46, A61K 33/28, A61P 31/12, A61P 31/16, A61P 31/22

(54) **ANTIVIRAL WIRKSAME PHARMAZEUTISCHE ZUSAMMENSETZUNG**
ANTIVIRALLY ACTIVE PHARMACEUTICAL COMPOSITION
COMPOSITION PHARMACEUTIQUE ANTIVIRALE ACTIVE

(30) Priorität: 28.06.2013 EP 13174235
(43) Veröffentlichungstag der Anmeldung: 04.05.2016
(73) Patentinhaber: Medice Arzneimittel Pütter GmbH & Co. KG, 58638 Iserlohn (DE)
(72) Erfinder: PUETTER, Sigurd, 58638 Iserlohn (DE); AMMER, Richard, 58644 Iserlohn (DE); SCHMIDBAUER, Michael, 42283 Wuppertal (DE)
(74) Vertreter: Isarpatent
(86) Internationale Anmeldenummer: PCT/EP2014/063671
(87) Internationale Veröffentlichungsnummer: WO 2014/207187

(56) Entgegenhaltungen:
- EP-A2- 0 513 878
- US-A1- 2002 031 509
- US-A1- 2011 105 423
- WHEELER A H ET AL: "THE EFFECT OF ATROPINE SULFATE ON THE COURSE OF INFLUENZA VIRUS INFECTION.", SCIENCE (NEW YORK, N.Y.) 8 DEC 1944, Bd. 100, Nr. 2606, 8. Dezember 1944 (1944-12-08), Seiten 523-524, XP002711776, ISSN: 0036-8075 in der Anmeldung erwähnt
- Z. YAMAZAKI ET AL: "Antiviral Effects of Atropine and Caffeine", JOURNAL OF GENERAL VIROLOGY, Bd. 50, Nr. 2, Oktober 1980 (1980-10), Seiten 429-431, XP055076401, ISSN: 0022-1317, DOI: 10.1099/0022-1317-50-2-429 in der Anmeldung erwähnt
- OZCELIK B ET AL: "Cytotoxicity, antiviral and antimicrobial activities of alkaloids, flavonoids, and phenolic acids", PHARMACEUTICAL BIOLOGY 2011 INFORMA HEALTHCARE GBR, Bd. 49, Nr. 4, April 2011 (2011-04), Seiten 396-402, XP009172049, ISSN: 1388-0209 in der Anmeldung erwähnt

## Beschreibung

### Gebiet der Erfindung

Die vorliegende Erfindung betrifft eine pharmazeutische Zusammensetzung umfassend Atropin oder ein Salz hiervon; Aconitin und Quecksilbercyanid zur Verwendung in einem Verfahren zur Behandlung einer Virusinfektion.

### Hintergrund der Erfindung

Viren sind die Haupterreger von Erkältungen. Unter ihnen nehmen Rhinoviren und Coronaviren, die für 50-70 % aller Atemwegsinfekte verantwortlich sind, die vorderen Plätze ein, gefolgt von Parainfluenzaviren, RSV, Adeno- und Enteroviren. Die Influenza, eine weitere akute Atemwegserkrankung, wird ebenfalls von Viren verursacht. Obendrein profitieren Herpes-simplex-Viren (HSV) von der einhergehenden Abwehrschwäche und können unter Umständen entsprechende Symptome wie Herpes labialis hervorrufen.

Während zur Behandlung von bakteriellen Infektionen eine Fülle von Antibiotika existieren, steht zur Behandlung virusbedingter Erkrankungen nur eine relativ kleine Anzahl von Virostatika gegen ein begrenztes Virenspektrum zur Verfügung. Die mit vielen Nebenwirkungen behafteten Antibiotika tragen verständlicherweise noch weniger zur Heilung der meist viral bedingten Erkältung bei, wirken sie doch lediglich gegen die möglichen, mit einer langwierigen Erkältung einhergehenden bakteriellen Sekundärinfektionen. Die oft übereilte Verordnung von Antibiotika im großen Stil ist zudem mit einer hohen Rate an Resistenzbildung assoziiert bzw. wirkt in einer großen Anzahl von Fällen überhaupt nicht. Wünschenswert sind somit effektive und verträgliche Alternativen.

Phytotherapeutika und Komplexhomöopathika können eine solche Alternative darstellen. In der Tat wurden und werden solche Alternativen immer wieder diskutiert, da sie gegenüber den synthetischen Heilmitteln Vorteile in Gestalt einer größeren therapeutischen Breite, einer besseren Verträglichkeit und weniger Arzneimittelwechselwirkungen besitzen sowie nicht der Gefahr der Resistenzbildung ausgesetzt sind.

In den letzten Jahren sind vermehrt Studien publiziert worden, in denen eine antivirale Wirkung von pflanzenbasierten Arzneimitteln nachgewiesen werden konnte. Das Komplexhomöopathikum Meditonsin® besteht aus den pflanzlichen Komponenten Atropin und Aconitin sowie dem Salz Quecksilbercyanid (Mercurius cyanatus) und wird bereits seit über 60 Jahren zur Behandlung von Entzündungen des Hals, Nasen- und Rachenraums verwendet. Klinische Untersuchungen bestätigen die Wirksamkeit bei der Behandlung von Entzündungen der oberen Atemwege und die gute Verträglichkeit.

EP 0 513 878 offenbart eine Lösung, die u.a. Cetylpyridiniumchlorid zu 0,10 g, Atropin-Hydrochlorid zu 0,004 g und Quecksilber-II-cyanid zu 0,004 g enthält (bezogen auf 100 ml). Eine antivirale Wirksamkeit der Lösung gegen Influenza-Viren wird beschrieben, genauer von Quecksilber-II-cyanid und Cetylpyridiniumchlorid.

Wheeler et al., "The Effect of Atropine Sulfate on the Course of Influenza Virus Infection", Science, 8.12.1944, Bd. 100, Nr. 2606 betrifft die antivirale Wirkung von Atropinsulfat (Influenza A-Viren). Die tatsächlich eingesetzte Konzentration an Atropin ist jedoch nicht angegeben. Zudem ist eine durchgängige Wirksamkeit von Atropin gegen Influenza A-Viren nicht belegt. Vielmehr hängt die Wirkung vom Zeitpunkt der intraperitonealen Verabreichung im Verhältnis zum Zeitpunkt der Virus-Inokulation der Versuchstiere ab.

Z. Yamazaki et al., "Antiviral Effects of Atropine and Caffeine", J. gen. Virol. (1980), 50, 429-431, offenbart die antivirale Wirkung von Atropin gegen Herpesviren und Influenzaviren. Die maximale Wirkung wird bei einer Atropin-Konzentration von 2 mM erreicht.

Özçelik et al., "Cytotoxicity, antiviral and antimicrobial activities of alkaloids, flavonoids, and phenolic acids", Pharmaceutical Biology, 2011; 49(4): 396-402, beschreibt u.a. die antivirale, antibakterielle, antimykotische Wirkung zahlreicher Alkaloide, sowie ihre Zytotoxizität. Eine antivirale Wirkung von Atropin im Konzentrationsbereich von 0,8-0,05 µg/ml, beispielsweise gegen HSV1, wird beschrieben.

US 2011/105423 betrifft Verbindungen, die die Replikation von RNA-Viren, wie Grippeviren, modulieren. Als Beispiel für derartige Verbindungen wird unter anderem Aconitin genannt.

US2002031509 bezieht sich auf eine pharmazeutische Zusammensetzung, die eine antimikrobiell wirksame Menge mindestens eines Schlangengifts umfasst. Auch wird eine Zusammensetzung beschrieben, die weiterhin einen Pflanzenextrakt enthält, beispielsweise Aconitin.

Die der vorliegenden Erfindung zugrunde liegenden Studien haben überraschenderweise ergeben, dass ein Gemisch aus Atropin, Aconitin und Quecksilbercyanid stark antivirale Eigenschaften aufweist. Besonderes Augenmerk wurde auf die Frage gelegt, ob, in welchen Konzentrationen und gegen welche Viren das Gemisch wirkt. Getestet wurde dabei gegen Erkältungs- und Herpesviren.

Die vorliegende Patentanmeldung betrifft somit die überraschende Erkenntnis, dass eine pharmazeutische Zusammensetzung, die Atropin oder ein Salz hiervon, Aconitin und Quecksilbercyanid umfasst in vorteilhafter Weiser zur Behandlung von Virusinfektionen eingesetzt werden kann.

### Zusammenfassung der Erfindung

Die vorliegende Erfindung zeigt die antiviralen Eigenschaften eines Gemisches der Wirkstoffe Atropin, Aconitin und Quecksilbercyanid in verschiedenen Konzentrationsbereichen. Dabei konnte nachgewiesen werden, dass das Gemisch die Aktivität aller getesteten Viren stark bis sehr stark hemmte.

Die vorliegende Erfindung betrifft somit eine pharmazeutische Zusammensetzung umfassend Atropin oder ein Salz hiervon, Aconitin und Quecksilbercyanid zur Verwendung in einem Verfahren zur Behandlung einer viralen Infektion, wobei die virale Infektion durch Herpesviren, Rhinoviren, RSV (humanes Respiratory-Syncytial-Virus) und/oder Enteroviren hervorgerufen wird.

### Kurze Beschreibung der Figuren

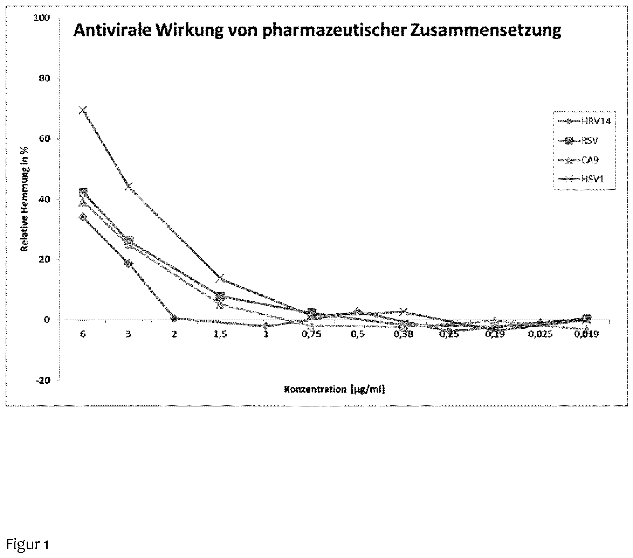

In Figur 1 ist die antivirale Wirkung der erfindungsgemäßen pharmazeutischen Zusammensetzung in Abhängigkeit von der Konzentration, gemessen als relative Hemmung in Prozent, angegeben. Die dazugehörigen Einzelwerte finden sich in Tabelle 3.

### Ausführliche Beschreibung der Erfindung

Die vorliegende Erfindung betrifft eine pharmazeutische Zusammensetzung umfassend oder im Wesentlichen bestehend aus a) Atropin oder einem Salz hiervon; b) Aconitin; und c) Quecksilbercyanid zur Verwendung in einem Verfahren zur Behandlung einer viralen Infektion, wobei die virale Infektion durch Herpesviren, Rhinoviren, RSV (humanes Respiratory-Syncytial-Virus) und/oder Enteroviren hervorgerufen wird.

Bevorzugt wird die virale Infektion durch HRV14 (humanes Rhinovirus Typ 14), HSV1 (Herpes-simplex-Virus Typ 1), und/oder CA9 (humanes Coxsackie-Virus Typ 9) hervorgerufen.

Der Begriff "pharmazeutische Zusammensetzung" wie hierin verwendet, umfasst insbesondere perorale Darreichungsformen, beispielsweise feste, halbflüssige oder flüssige Zusammensetzungen zur oralen Verabreichung. Eine wässrig/ethanolische Lösung zur tropfenweisen Verabreichung hat sich hierbei als besonders vorteilhaft herausgestellt. Beispielsweise werden Gemische von Ethanol 94 % (g/g), Glycerol 85 % sowie gereinigtem Wasser im Gewichtsverhältnis 5:10:85 besonders vorteilhaft eingesetzt.

Alternativ sind jedoch auch andere Darreichungsformen möglich, beispielsweise feste perorale Darreichungsformen wie Pulver, Tabletten oder Kapseln. Die Herstellung erfolgt insbesondere nach den Regeln für die Herstellung homöopathischer Arzneimittel gemäß Homöopathischem Arzneibuch 2012 (HAB 2012), amtliche Ausgabe. Beispielsweise können Verreibungen nach Vorschrift des HAB hergestellt werden, bei denen die Ausgangsstoffe stufenweise mit Lactose-Monohydrat verrieben werden. Alternativ können Tabletten aus den entsprechenden Verreibungen verpresst werden. Hierbei können begrenzte Zusätze von Magnesiumstearat oder Calciumbehenat als Gleitmittel und von Stärke als Zerfallsbeschleuniger verwendet werden. Im Falle der Granulation können wässrige Lactose-Lösung, Ethanol und geeignete Konzentrationen von Stärkekleistern eingesetzt werden.

Zudem können Streukügelchen nach der HAB-Vorschrift 10 hergestellt werden, wobei Zuckerkügelchen mit 1/100 Massenteilen der betreffenden Dilution gleichmäßig befeuchtet und an der Luft getrocknet werden.

Neben peroralen Arzneiformen kann die pharmazeutische Zusammensetzung der vorliegenden Erfindung Parenteralia, d.h. flüssige Verdünnungen zur Injektion sowie flüssige Einreibungen sowie Salben, Suppositorien, Augentropfen, Nasentropfen umfassen, die nach den HAB-Vorschriften 11 - 15 sowie 45 hergestellt wurden. Wie oben ausgeführt, handelt es sich bei der vorliegenden pharmazeutischen Zusammensetzung in der am meisten bevorzugten Ausführungsform um eine perorale Lösung, die nach den Vorschriften des Homöopathischen Arzneibuchs hergestellt wurde.

Die Formulierung "besteht im Wesentlichen aus" bedeutet, dass die pharmazeutische Zusammensetzung diese Bestandteile als wirksame Bestandteile enthält. Sie kann jedoch weiterhin ein oder mehrere Lösungsmittel, Hilfsstoffe etc. enthalten, die zwar zur Herstellung einer pharmazeutischen Zusammensetzung notwendig sind, jedoch nicht oder nicht wesentlich zur pharmakologischen Wirksamkeit beitragen.

Einer der Bestandteile der pharmazeutischen Zusammensetzung ist Atropin. Atropin ist ein Tropan-Alkaloid, bei dem es sich um ein Racemat aus den Isomeren (R)- und (S)-Hyoscyamin entsprechend der folgenden Strukturformel handelt. Die (R)-Form ist oben, die (S)-Form unten angegeben:

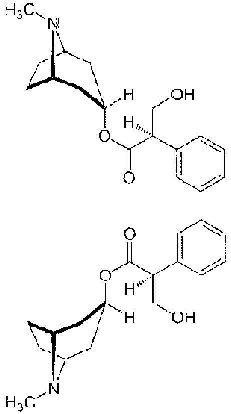

Atropin gehört zu den Parasympatholytika und konkurriert an den muskarinischen Rezeptoren des Parasympathikus mit dem Neurotransmitter Acetylcholin. Die körperlichen Wirkungen der Einnahme von Atropin sind wie folgt: Beschleunigung der Herzfrequenz, Weitstellung der Bronchien, verminderte Speichelbildung u.v.a. Darüber hinaus wurden weitere, jedoch seltene Anwendungsgebiete beschrieben, beispielsweise die Anwendung bei Krämpfen des Magen-Darmtraktes, bei erschwerter Blasenentleerung sowie Harninkontinenz. Eine Verwendung als Antivirusmittel ist jedoch bislang nicht beschrieben worden.

Bei dem erfindungsgemäß eingesetzten Salz des Atropins handelt es sich üblicherweise um Atropinsulfat, beispielsweise Atropinsulfat-Monohydrat.

In der erfindungsgemäßen Zusammensetzung wird Atropin oder sein Salz in einer Konzentration zwischen 1 bis 100 µg/ml, bevorzugt 1 bis 10 µg/ml, bevorzugter 2 bis 7 µg/ml und am meisten bevorzugt 3,13 bis 6,25 µg/ml eingesetzt. Erfindungsgemäß hat sich herausgestellt, dass, falls Atropinsulfat in Kombination mit den weiteren Bestandteilen der pharmazeutischen Zusammensetzung verwendet wird, von einer niedrigeren Dosierung von Atropinsulfat auszugehen ist (siehe unten). Mit anderen Worten kann bei niedrigerer Konzentration ein wenigstens vergleichbarer, möglicherweise sogar deutlich besserer anti-viraler Effekt erreicht werden.

Als weiterer Bestandteil der pharmazeutischen Zusammensetzung kommt Aconitin zum Einsatz. Aconitin ist ein Diterpenoid-Alkanoid, das die Inaktivierung des spannungsabhängigen Natriumkanals verlangsamt und dadurch den Einstrom von Natriumionen während des Aktionspotentials der Nerven verlängert. Es wirkt insofern zunächst zentral wie peripher auf motorische wie sensible Nerven zunächst erregend gefolgt von einer Lähmung. Kardiale Auswirkungen können Arrhythmien, Bradykardie sowie diastolischen Herzstillstand mit sich bringen. Die *dosis letalis* von Aconitin liegt bei Erwachsenen bei ca. 5 mg. Aconitin weist folgende Strukturformel auf:

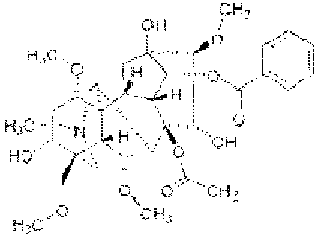

Die chemische Summenformel lautet C₃₄H₄₇NO₁₁.
Aconitin wird als wirksamer Bestandteil der erfindungsgemäßen Zusammensetzung in einer Konzentration zwischen 0,05 - 100 µg/ml, beispielsweise, 0,5 - 100 µg/ml, bevorzugt 0,05 - 5 µg/ml, bevorzugter 0,5 - 2 µg/ml und am meisten bevorzugt 0,16 - 1,56 µg/ml Aconitin eingesetzt. Auch hier hat sich herausgestellt, dass in Kombination mit den beiden weiteren wirksamen Bestandteilen der erfindungsgemäßen pharmazeutischen Zusammensetzung, nämlich Quecksilber-II-cyanid und Atropin, eine synergistische Wirkung erzielt werden kann, d.h. ein überadditiver antiviraler Effekt bei gleicher oder niedrigerer Dosierung der Einzelkomponenten.

Der dritte Bestandteil der erfindungsgemäßen pharmazeutischen Zusammensetzung ist Quecksilbercyanid (Hg(CN)₂). Quecksilbercyanid ist eine hochgiftige Verbindung, die lediglich in der Homöopathie zur Behandlung von Diphterie, schwerer Angina und Erkältungen verwendet wird.

Quecksilbercyanid wird als Komponente der erfindungsgemäßen Zusammensetzung in Konzentrationen zwischen 0,001 bis 10 µg/ml, bevorzugt 0,001 bis 0,1 µg/ml, bevorzugter 0,001 bis 0,01 µg/ml, am meisten bevorzugt 0,003 - 0,006 µg/ml Quecksilbercyanid in Lösung eingesetzt.

Wie oben angesprochen umfasst die pharmazeutische Zusammensetzung der vorliegenden Erfindung eine Kombination aus Atropin (oder einem Salz hiervon), insbesondere Atropinsulfat, sowie Aconitin und Quecksilbercyanid. Für eine derartige Zusammensetzung hat es sich als vorteilhaft herausgestellt, die Konzentration der jeweiligen Einzelbestandteile niedriger anzusetzen als bei der jeweiligen Einzelsubstanz. So kann die erfindungsgemäße pharmazeutische Zusammensetzung, beispielsweise in Form einer wässrig/ethanolischen Lösung, ungefähr 1 - 10, vorzugsweise 5 µg/ml Atropinsulfat, ungefähr 0,5 - 5, vorzugsweise 1, µg/ml Aconitin und ungefähr 0,001 - 0,1 µg/ml, vorzugsweise 0,004 µg/ml, Quecksilbercyanid enthalten.

Eine vorteilhafte Zusammensetzung gemäß der vorliegenden Erfindung umfasst eine Gesamtkonzentration von ca. 3-9 µg/ml der vorgenannten Inhaltsstoffe. Wie aus Figur 1 ersichtlich ist zeigen sich bei einer Gesamtkonzentration von 6 µg/ml die besten Ergebnisse bezüglich der antiviralen Wirksamkeit, jedoch wurden auch mit niedrigeren Werten, z.B. einer Gesamtkonzentration von 3 µg/ml noch hervorragende Hemmwerte erzielt.

Eine besonders bevorzugte Zusammensetzung umfasst eine Lösung aus Ethanol, Glycerol und Wasser, die ungefähr 5 µg/ml Atropinsulfat, 1 µg/ml Aconitin sowie 0,004 µg/ml Quecksilbercyanid enthält. Eine derartige Zusammensetzung ist bereits, wie in der Einleitung geschildert, bekannt und wird unter der Bezeichnung "Meditonsin®" seit Jahrzehnten erfolgreich auf dem europäischen Markt vertrieben. Erstaunlicherweise hat sich herausgestellt, dass die Verwendung der Dreierkombination gegenüber der antiviralen Aktivität der Einzelkomponenten eine synergistische Wirkung entfaltet. Wie sich den Beispielen entnehmen lässt, ist die so genannte "Meditonsin"-Mischung insbesondere gegen RSV, CA9, HRV14 und HSV1 stark bis sehr stark antiviral wirksam. Dies insbesondere unter Berücksichtigung der Tatsache, dass die in der Mischung eingesetzten Konzentrationen der Einzelkomponenten niedriger sind als die jeweils für die Einzelversuche eingesetzten Mengen.

Tabelle 2 zeigt eine vergleichende Darstellung der antiviralen Wirkung zwischen den Substanzen Atropinsulfat, Aconitin und Quecksilberzyanid sowie deren Mischung. Es ist die prozentuale Plaquehemmung als Messergebnis angegeben. Beispielsweise beträgt die prozentuale Plaquehemmung für das Gemisch aus 5 µg/ml Atropinsulfat, 1 µg/ml Aconitin sowie 0,004 µg/ml Quecksilbercyanid bezogen auf HSV1 69,53%. Dies ist ein überraschend hoher Wert, wenn man die Plaquehemmung der Einzelsubstanzen berücksichtigt: diese beträgt bei Atropinsulfat ca. 35%, für Aconitin ca. 8% und für Quecksilbercyanid sogar einen leicht negativen Wert. Verglichen mit dem aus den Einzelmessungen zu erwartenden Wert ist die Hemmwirkung des Gemisches auf HSV1 beinahe doppelt so hoch. Davon abgesehen ist eine Plaquehemmung von beinahe 70% ein hervorragender Wert, der das hohe therapeutische Potential der eingesetzten Mischung verdeutlicht.

Ähnlich erstaunliche Resultate sind Tabelle 2 in Hinblick auf CA9, RSV und HRV14 zu entnehmen. Auch hier ergibt sich eine überadditive Wirkung der Einzelkomponenten im Gemisch, die ungefähr bei einem Faktor 2 gegenüber den Einzelsubstanzen anzusetzen ist.

Die pharmazeutische Zusammensetzung der vorliegenden Erfindung (Gemisch) wird in einer täglichen Gesamtdosis von ungefähr 20 µg/ml Atropinsulfat, 4 µg/ml Aconitin und 0,016 µg/ml Quecksilbercyanid an den erwachsenen Patienten verabreicht. Abweichungen von ca. ± 30 % von der angegebenen Menge sind hierbei tolerabel.

Mit anderen Worten werden einem erwachsenen Patienten ca. 2 - 6 ml der pharmazeutischen Zusammensetzung pro Tag verabreicht, wobei die Zusammensetzung die oben angegebenen Konzentrationen aufweist. Die Dosierung halbiert sich für Kinder zwischen dem 6. und 12. Lebensjahr, wird für Kleinkinder ab dem 1. Lebensjahr noch weiter reduziert und beträgt ungefähr 25 % für Säuglinge ab dem 7. Lebensmonat.

Die vorliegende Erfindung wird nunmehr unter Bezugnahme auf die nachfolgenden Ausführungsbeispiele erläutert, in denen die Effekte der erfindungsgemäßen Zusammensetzung dargelegt werden.

### Beispiele:

### Zweck/Ziel

Die im Nachfolgenden beschriebene antivirale Testung hatte zum Ziel, die antivirale Wirkung von Atropinsulfat, Aconitin und Quecksilberzyanid sowie einer Mischung aus diesen drei Substanzen zu untersuchen.

### Teststrategie

Die antivirale Testung von Atropinsulfat, Aconitin und Quecksilberzyanid sowie einer Mischung aus diesen drei Substanzen teilt sich in zwei Phasen. In der ersten Phase wird eine Zytotoxizitätsprüfung der Testsubstanzen in verschiedenen Verdünnungsstufen durchgeführt, um herauszufinden, ab welcher Verdünnungsstufe kein zytotoxischer Effekt von den Testsubstanzen auf die als Virenträger dienenden, nicht virusinfizierten Zelllinien ausgeübt wird. In der zweiten Phase wird die antivirale Wirkung der Testsubstanzen in verschiedenen Verdünnungsstufen auf die im Test verwendeten Viren untersucht, beginnend mit der Testsubstanzverdünnung, ab der kein zytotoxischer Effekt auf die Zelllinien ausgeübt wird.

### Material

### Prüfmuster

Von den Rohstoffen Atropinsulfat (AT), Aconitin (AC) und Quecksilberzyanid (QC) wurden die Prüfmuster für die Toxizitätsprüfung und der anschließenden Prüfung auf antivirale Wirkung gemäß nachfolgender Tabelle hergestellt.

| **Lfd. Nr.** | **Prüfsubstanz** | **Einwaage/Löslichkeit in Ethanol** | **Konzentration** | **Toxizitätsprüfung erste Verdünnung: 1:10 = höchste Konzentration im Test** |
|---|---|---|---|---|
| Nr. 1 | Atropinsulfat (AT) | 100 mg/10ml / 80% Ethanol | 10 mg/ml / 80% Ethanol | 1 mg/ml / 8% Ethanol |
| Nr. 2 | Aconitin (AC) | 100 mg/10ml / 80% Ethanol | 10 mg/ml / 80% Ethanol | 1 mg/ml / 8% Ethanol |
| Nr. 3 | Quecksilberzyanid (QC) | 10 mg/ml / 43% Ethanol | 10 mg/ml / 43% Ethanol | 1 mg/ml / 4,3% Ethanol |
| Nr. 4 | Mischung Nr. 1-3 | AT: 0,05 mg in 5 ml / 0,008% Ethanol | | 6,004 µg/ml / 0,004% Ethanol |
| | | AC: 0,01 mg in 1 ml / 0,008% Ethanol | | |
| | | QC: 0,00004 mg in 4ml / 0,000043% Ethanol | | |

### Zellen

- Humane Epithelzellen (HEp-2-Zellen) für Infektionen mit RSV und HSV1
- Buffalo-Green-Monkey-Zellen (BGM-Zellen) für Infektionen mit CA9
- HeLa-Zellen für Infektionen mit HRV14 (Die HeLa-Zelllinie stammt von menschlichen Epithelzellen eines Zervixkarzinoms ab)

### Viren

- Humanes Respiratory Syncytial Virus, Stamm Long (RSV)
- Humanes Coxsackievirus Typ 9 (CA9)
- Humanes Herpesvirus Typ 1 (HSV1)
- Humanes Rhinovirus Typ 14 (HRV14, Major group)

### Referenz-Kontrolle und laborinterne Standards

- Ribavirin (Viracole®), ICN Pharmaceuticals, gegen RSV (5 µg/ml)
- Laborinterne Standards (L-Std) gegen Infektionen mit CA9 (L-Std., 6 µg/ml)
- Aciclovir (2,5 µg/ml) gegen HSV1
- Oxymetazolinhydrochloride (OMZ, 10µg/ml) gegen HRV14

### Zytotoxizitätstest zum Ausschluss einer möglichen Intoxikation von Zellkulturen

Die Zytotoxizität der Prüfmuster wurde mittels des MTT-Tests (Stoffwechselprüfung) untersucht. Die Testansätze für die Stoffwechselleistungsprüfung im MTT-Test wurden in 96-Loch-Mikrotiterplatten diejenigen Testansätze für die mikroskopische Analyse in 24-Loch-Mikrotiterplatten.

### MTT-Test

### Prinzip

Die Bestimmung der Stoffwechselleistung im MTT-Test erfolgt über die quantitative Analyse der mitochondrialen Enzymaktivität. Der kolorimetrische Test nach Mosmann (Mosman 1983) beruht auf der Umsetzung des farblosen MTT-Salzes (Dimethylthiazol-diphenylterazolium-Bromid) in blaue, wasserunlösliche Formazankristalle durch mitochondriale Dehydrogenasen. Es kann hierbei eine direkte Korrelation zwischen der Vitalität bzw. der Stoffwechselleistung und der Enzymaktivität der Zellen gemessen werden.

### Prüfprotokoll

Nach dem Lösen und dem Vorbereiten der Prüfmuster gemäß Abschnitt 0 erfolgte die Toxizitätsprüfung. Es folgten bei allen Prüflösungen weitere log2- bzw. log10-Verdünnungsabstufungen. Alle Testsubstanzverdünnungen wurden in Parallelproben zu wachsenden BGM-, HEp-2- und HeLa-Zellen gegeben. Danach wurden die behandelten Zellkulturen für insgesamt 3 Tage bzw. 5 Tage bei 37°C und 5% CO₂ inkubiert. Als Kontrollen wurden Zellen mit Medium allein (unbehandelte Kontrolle) bzw. mit 8-, 4-, 2-, 1- und 0,004% Ethanol im Medium (Ethanol-Kontrolle) mitgeführt.

### Auswertung

Die Analyse im MTT-Test erfolgte am Tag 1 und Tag 3 bzw. am Tag 1 und Tag 5 nach Substanzzugabe. Dafür wurden die behandelten Zellen für weitere 2 bis 4 Stunden mit der MTT-Lösung inkubiert und nach Solubilisierung der Formazankristalle in Dimethylsulfoxid (DMSO) die optische Dichte (OD) der Zellkulturüberstände im Photometer bei 570 nm bestimmt (RF690nm). Für die Auswertung gilt: je mehr Zellen geschädigt sind, desto weniger Formazankristalle wurden gebildet und desto niedriger ist die OD. Die mikroskopische Beurteilung einer veränderten Zellmorphologie erfolgte am Tag 3 bzw. Tag 5 nach Substanzzugabe über das Kriterium einer unveränderten Zellmorphologie bzw. über vergrößerte Zellen mit Vakuolisierung bis hin zu einem abgelösten Zellrasen.

### Berechnung der prozentualen relativen Vitalität (Statistik Excel 2000)

Der OD-Werte der unbehandelten Kontrollen wurden als 100% Vitalität definiert. Die OD-Werte der Testansätze wurden entsprechend als Prozentwerte (%Toxizität) angegeben. Hierbei war es möglich, über mehrere Parallelproben die 50%ige hemmende Dosis (IC₅₀) in Dosiswirkungskurven der jeweiligen Prüfmuster zu bestimmen bis hin zu der Konzentration, die keinerlei testsubstanzbedingte Beeinträchtigung aufwies für den Einsatz in die antiviralen Prüfungen.

### Mikroskopischer Befund über die Analyse der Zellmorphologie

In Parallelansätzen zur Bestimmung der Stoffwechselleistung im enzymatischen MTT-Test wurde der Einfluss der Prüfsubstanzen auf die Zellen über die mikroskopische Beurteilung der Zellmorphologie am Tag 3 bzw. Tag 5 nach Testansatz über folgende Kriterien bestimmt:
- keine veränderte Zellmorphologie (-),
- Zellen mit leichter Vakuolenbildung (+),
- vergrößerte Zellen und massive Vakuolenbildung (++),
- abgekugelte Zellen (+++),
- ein zerstörter Zellrasen. (++++),
- ein fixierter Zellrasen durch mögliche oberflächenaktive Inhaltsstoffe (F) und
- durch Niederschlag von Mediumkomponenten auf dem Zellrasen (N).

### Prüfung einer antiviralen Wirkung

Die quantitative Analyse der antiviralen Aktivität erfolgte in Plaquereduktionsassays und in virusspezifischen Enzymimmunoassays.

### Verwendete Tests

### Plaquereduktionsassay (PFU/ml)

### Prinzip

Für die Durchführung der Plaquereduktionsassays wurden konfluent gewachsene Zellen (Zellrasen) mit einer definierten Viruslösung (M.O.I = multiplicity of infection) infiziert. Nach einer einstündigen Inkubation wurde das Virusinokulum abgezogen und die Zellen (Zellrasen) gewaschen. Anschließend wurden die virusinfizierten Zellen mit den physiologischen Substanzkonzentrationen sowie dem Zusatz einer festen Mediumkomponente (Agarose oder Methylzellulose) überlagert und weiter kultiviert. Durch die feste Komponente im überlagerten Medium wird die Infektionsfläche begrenzt, so dass ein Herd von virusinfizierten Zellen ("Plaque") entsteht. Die jeweiligen Testansätze wurden solange kultiviert, bis in den unbehandelten Virus-Kontrollen die eingestellte Plaquezahl (M.O.I.) mikroskopisch zu beobachten war.

### Auswertung

Durch Fixierung und Färbung des Zellrasens können die Virusplaques als helle Höfe im dunkel gefärbten Zellrasen sichtbar gemacht werden. Die Bestimmung der Plaquezahl erfolgte mit Hilfe eines Bildverarbeitungssystems.

### Berechnung der prozentualen Hemmung (%Hemmung, Statistik Excel 2000)

Die Plaquezahl der unbehandelten Kontrolle wurde als 100-prozentige Infektion definiert. Demgegenüber wurden die Plaquezahlen der jeweiligen Testansätze ausgewertet, so dass hemmende Effekte der zu analysierenden Substanzen als prozentuale Substanzhemmung (%Hemmung) gezeigt werden konnten.

### Enzymimmunoassay (ELISA) (Analyse der Virusproduktion)

### Prinzip

Bei der hier angewandten Sandwich-ELISA-Technik sind die Mikroteststreifen mit Antikörpern gegen die spezifischen Viren beschichtet. Die im Zellkulturüberstand der infizierten Zelllinien befindlichen Viren binden an die festphasenfixierten Antikörper. Um die Reaktion sichtbar zu machen, setzt man mit dem Enzym Peroxidase markierte erregerspezifische Nachweisantikörper ein, die gegen die entsprechenden Viren gerichtet sind. Gibt man in einem weiteren Schritt das Substrat/Chromogen sowie Wasserstoffperoxid und Tetramethylbenzidin hinzu, so erfolgt eine Enzym-Substrat-Chromogen-Reaktion zu einem blauen Farbstoff. Die Intensität der Färbung wird photometrisch (Extinktion) im Photometer bei einer Wellenlänge 450 nm (OD450) bestimmt; sie ist dem Virusantigen-Gehalt proportional.

### Durchführung

Die Analyse der Virusproduktion bei infizierten und behandelten Zellen erfolgte über die Bestimmung viraler Proteine (konservierte virusspezifische Antigene bei RSV) im ELISA. Hierzu wurden die konfluent gewachsenen virussensitiven Zellen (Zellrasen) mit einer definierten Viruslösung (M.O.I.) infiziert. Nach einstündiger Inkubation wurde das Virusinokulum abgezogen und der infizierte Zellrasen gewaschen. Im Anschluss erfolgte die Zugabe der physiologischen Substanzkonzentrationen. Die jeweiligen Testansätze wurden solange kultiviert, bis in den unbehandelten Virus-Kontrollen mikroskopisch ein 70- bis go-prozentiger CPE zu beobachten war. Die neusynthetisierten Viren befanden sich in diesem Stadium im Zellkulturüberstand. Es folgte daraufhin die Abnahme der virushaltigen Zellkulturüberstände für die Analyse der viralen Proteine im ELISA.

### Berechnung der prozentualen Hemmung (% Hemmung, Statistik Excel 2000)

Die im Photometer nach den Enzymreaktionen im ELISA ermittelten Extinktionswerte (OD492nm) der unbehandelten Kontrollen wurden als 100-prozentige Infektion definiert. Demgegenüber wurde die OD der jeweiligen Testansätze ausgewertet, so dass hemmende Effekte der zu analysierenden Substanzen als prozentuale Substanzhemmung (%Hemmung) gezeigt werden konnten.

### Testprotokoll

Die virusinfizierten Zellen wurden direkt nach Infektion mit den Prüfsubstanzen behandelt. Hierfür wurden die virussensitiven Zellen mit den jeweiligen vorbereiteten Viruslösungen infiziert (HEp-2: RSV, HSV1; BGM: CA9; HeLa: HRV14). Für die antivirale Prüfung erfolgten die jeweiligen Virusinfektionen mit einer mittleren Infektionsdosis (M.O.I. multiplicity of infection) von 0,0004 (RSV, HSV1, HRV14) bzw. 0,0003 (CA9).

Die Zugabe der Prüflösungen in verschiedenen Verdünnungsstufen zu den infizierten Zellen erfolgte nach Überprüfung einer möglichen Intoxikation in den Zellvitalitätstesten. Die Verdünnungsreihe begann mit nicht zelltoxischen Konzentrationen von 50 µg/ml (Prüfmuster Nr.1 und Nr.2), 2 µg/ml (Prüfmuster Nr.3) sowie 6 µg/ml (Prüfmuster Nr.4). Die Verdünnung erfolgte in log2- bzw. log10-Abstufungen. Die Tabelle 1 zeigt die in der antiviralen Prüfung eingesetzten Konzentrationen der Prüfmuster.

Die quantitative Analyse einer antiviralen Aktivität erfolgte im Plaquereduktionsassay durch das Auszählen der Virusplaques am Tag 3 bis Tag 5 nach Testansatz mit Hilfe eines Bildverarbeitungssystems. Zudem wurden bei RSV neben den Experimenten zur Virusplaqueanalyse in analog durchgeführten Testansätzen die Zellkulturüberstände der infizierten und behandelten Zellen auf den Gehalt neusynthetisierter viraler Proteine in Enzymimmunoassays (ELISA) geprüft.

Zur Verifizierung des Testsystems wurden antivirale Wirkstoffe eingesetzt, Ribavirin (Viracole®) gegen RSV, das Aciclovir (Zovirax®) gegen HSV1 und Oxymetazolinhydrochlorid (OMZ) gegen HRV14. Eine Konzentration von 10 µg OMZ/ml, 5 µg Ribavirin pro ml bzw. 2,5 µg Aciclovir pro ml bei eingesetzter M.O.I. von 0,0004 erbrachten im Mittel eine Reduktion der Infektiosität von etwa 50-60%. Im Falle von CA9 wurden laborinterne Standards mitgeführt. Die laborinternen Standards wurden dabei so eingestellt, dass eine 50- bis 70%ige Reduktion der Infektiosität nachgewiesen werden konnte.

Im Weiteren wurden im Testsystem neben den Positiv-Kontrollen das ethanolische Lösungsmittel der jeweiligen Prüflösungen in den Endkonzentrationen 0,8% (No.1, NO.2), 0,0043% (No.3) bzw. 0,048% (No.4) EtOH im Medium (MEM) mitgeführt. Es konnte dabei kein Einfluss auf die Virusplaqueentwicklung bzw. auf die Infektiosität der Viren festgestellt werden.

**Tabelle 1: In der antiviralen Prüfung eingesetzte Konzentrationen der Prüfmuster**

| **Prüfmuster** | **Konzentrationen [µg/ml] für die antivirale Prüfung** | | | | | | | | | | | | | | | | |
|---|---|---|---|---|---|---|---|---|---|---|---|---|---|---|---|---|---|
| No.1 AT | 100 | 50 | 25 | 12,5 | 6,25 | 3,125 | 1,56 | 0,78 | 0,156 | 0,078 | 0,0078 | | | | | | |
| No.2 AC | 100 | 50 | 25 | 12,5 | 6,25 | 3,125 | 1,56 | 0,78 | 0,156 | 0,078 | 0,0078 | | | | | | |
| No.3 QC | 2 | 1 | 0,5 | 0,25 | 0,125 | 0,08 | 0,06 | 0,04 | 0,02 | 0,01 | 0,006 | 0,005 | 0,0025 | 0,0012 | 0,0006 | 0,00006 | 0,000006 |
| No.4 AT/AC/QC | 6,004 | 3,002 | 2,000 | 1,500 | 1,000 | 0,750 | 0,500 | 0,380 | 0,250 | 0,19 | 0,025 | 0,02 | | | | | |

### Synergieeffekt von Mischung aus Atropinsulfat, Aconitin und Quecksilberzyanid

**Tabelle 2: Vergleichende Darstellung der antiviralen Wirkung zwischen den Substanzen Atropinsulfat, Aconitin und Quecksilberzyanid sowie deren Mischung. Hier ist die prozentuale Plaquehemmung als Messergebnisse angegeben. Die Messergebnisse der einzelnen Substanzen sind für den Konzentrationsbereich angegeben, der demjenigen entspricht, wie er in der Mischung vorlag.**

| **Virus** | **Atropinsulfat (3,125-6,25 µg/ml)** | **Aconitin (0,156-1,56 µg/ml)** | **Quecksilberzyanid (0,0025-0,006 µg/ml)** | **Mischung (6,004 µg/ml)** |
|---|---|---|---|---|
| HSV1 | 19,52/41,90 | 3,17/11,22 | 0,00/-1,39 | 69,53 |
| CA9 | 7,63/12,85 | 8,40/10,69 | -2,32/0,00 | 39,16 |
| RSV | 7,74/12,36 | 9,01/16,56 | -3,20/5,33 | 42,41 |
| HRV14 | 11,30/14,12 | -1,14/2,84 | 15,48*) | 34,04 |

| | | | | |
|---|---|---|---|---|
| *) Es wurde nur ein Wert gemessen. | | | | |

In Tabelle 2 sind die Werte für die antivirale Wirkung, gemessen anhand der prozentualen Hemmung der viralen Aktivität, von Atropinsulfat, Aconitin und Quecksilberzyanid sowie von der Mischung dieser drei Substanzen. Bei den einzelnen Substanzen sind nur diejenigen Messwerte angegeben, die gemessen wurden in den Konzentrationsbereichen, welche denjenigen der einzelnen Substanzen in der Mischung entsprachen. In der Mischung lagen folgende Substanzkonzentrationen vor: 5 µg/ml Atropinsulfat, 1 µg/ml Aconitin und 0,004 µg/ml Quecksilberzyanid.

Die Tabelle 2 zeigt deutlich, zum Einen dass die Gesamtwirkung der Mischung höher ist als die Wirkung der Substanzen als einzelne und zum anderen die antivirale Wirkung der Mischung nicht auf einer Addition der antiviralen Wirkung der einzelnen Substanzen beruht, sondern auf einem synergistischen Effekt. Zum Beispiel müssten bei Vorliegen eines additiven Effektes im Falle der antiviralen Wirkung auf HSV1 die Messwerte der Mischung zwischen 22,69 und 51,73 liegen, was aber nicht der Fall ist. Tatsächlich zeigt die Mischung einen Wert von 69,53.

**Tabelle 3: In-vitro-Test der antiviralen Wirkung von pharmazeutischer Zusammensetzung in Abhängigkeit von der Konzentration, gemessen als relative Hemmung in Prozent (siehe Figur 1)**

| **Konzentrationen [µg/ml]** | | | | | | | | | | | | |
|---|---|---|---|---|---|---|---|---|---|---|---|---|
| **Viren** | **6** | **3** | **2** | **1,5** | **1** | **0,75** | **0,5** | **0,375** | **0,25** | **0,19** | **0,025** | **0,019** |
| HRV1 4 | 34,04 | 18,62 | 0,53 | - | -2,13 | - | 2,66 | - | -3,72 | - | -1,06 | - |
| RSV | 42,41 | 26,18 | - | 7,85 | - | 2,36 | - | -1,57 | - | -2,36 | - | 0,52 |
| CA9 | 39,16 | 24,92 | - | 5,18 | - | -1,94 | - | -2,27 | - | -0,32 | - | -3,24 |
| HSV1 | 69,53 | 44,23 | - | 13,76 | - | 1,47 | - | 2,70 | - | -3,44 | - | 0,00 |

## Patentansprüche

1. Pharmazeutische Zusammensetzung umfassend
a) Atropin oder ein Salz hiervon;
b) Aconitin; und
c) Quecksilbercyanid;
zur Verwendung in einem Verfahren zur Behandlung einer viralen Infektion, wobei die virale Infektion durch Herpesviren, Rhinoviren, RSV (humanes Respiratory-Syncytial-Virus) und/oder Enteroviren hervorgerufen wird.

2. Pharmazeutische Zusammensetzung zur Verwendung in einem Verfahren zur Behandlung einer viralen Infektion nach Anspruch 1, wobei die virale Infektion durch HRV14 (humanes Rhinovirus Typ 14), HSV1 (Herpes-simplex-Virus Typ 1), und/oder CA9 (humanes Coxsackie-Virus Typ 9) hervorgerufen wird.

3. Pharmazeutische Zusammensetzung zur Verwendung in einem Verfahren zur Behandlung einer viralen Infektion nach einem oder mehreren der vorhergehenden Ansprüche, wobei die Zusammensetzung als wässrig/ethanolische Lösung vorliegt und ungefähr 5 µg/ml Atropinsulfat, ungefähr 1 µg/ml Aconitin und ungefähr 0,004 µg/ml Quecksilbercyanid enthält.

4. Pharmazeutische Zusammensetzung zur Verwendung in einem Verfahren zur Behandlung einer viralen Infektion nach Anspruch 1 oder 2, wobei die Zusammensetzung zwischen 1-10 µg/ml, bevorzugt 2-7 µg/ml, am meisten bevorzugt 3,13-6,25 µg/ml Atropinsulfat enthält.

5. Pharmazeutische Zusammensetzung zur Verwendung in einem Verfahren zur Behandlung einer viralen Infektion nach einem oder mehreren der Ansprüche 1-2, wobei die Zusammensetzung zwischen 0,05-5 µg/ml bevorzugt 0,5-2 µg/ml, am meisten bevorzugt 0,16-1,56 µg/ml Aconitin enthält.

6. Pharmazeutische Zusammensetzung zur Verwendung in einem Verfahren zur Behandlung einer viralen Infektion nach einem oder mehreren der Ansprüche 1-2, wobei die Zusammensetzung zwischen 0,001 und 0,1 µg/ml, bevorzugt 0,001-0,01 µg/ml, am meisten bevorzugt 0,003-0,006 µg/ml Quecksilbercyanid enthält.

7. Pharmazeutische Zusammensetzung zur Verwendung in einem Verfahren zur Behandlung einer viralen Infektion nach einem oder mehreren der vorhergehenden Ansprüche, wobei die Zusammensetzung in einer täglichen Dosis von ungefähr 20 µg/ml Atropinsulfat, 4 µg/ml Aconitin und 0,016 µg/ml Quecksilbercyanid verabreicht wird.

## Claims

1. Pharmaceutical composition comprising
a) atropine or a salt thereof;
b) aconitine; and
c) mercury cyanide;
for use in a method for treating a viral infection, wherein the viral infection is brought about by herpes viruses, rhinoviruses, RSV (human respiratory syncytial virus) and/or enteroviruses.

2. Pharmaceutical composition for use in a method for treating a viral infection according to claim 1, wherein the viral infection is brought about by HRV₁₄ (human rhinovirus type 14), HSV₁ (herpes simplex virus type 1) and/or CA₉ (human coxsackievirus type 9).

3. Pharmaceutical composition for use in a method for treating a viral infection according to one or more of the preceding claims, wherein the composition is present as an aqueous / ethanolic solution and contains approximately 5 µg/ml atropine sulphate, approximately 1 µg/ml aconitine and approximately 0.004 µg/ml mercury cyanide.

4. Pharmaceutical composition for use in a method for treating a viral infection according to either claim 1 or claim 2, wherein the composition contains 1-10 µg/ml, preferably 2-7 µg/ml, most preferably 3.13-6.25 µg/ml atropine sulphate.

5. Pharmaceutical composition for use in a method for treating a viral infection according to one or more of claims 1-2, wherein the composition contains 0.05-5 µg/ml, preferably 0.5-2 µg/ml, most preferably 0.16-1.56 µg/ml aconitine.

6. Pharmaceutical composition for use in a method for treating a viral infection according to one or more of claims 1-2, wherein the composition contains between 0.001 and 0.1 µg/ml, preferably 0.001-0.01 µg/ml, most preferably 0.003-0.006 µg/ml mercury cyanide.

7. Pharmaceutical composition for use in a method for treating a viral infection according to one or more of the preceding claims, wherein the composition is administered in a daily dose of approximately 20 µg/ml atropine sulphate, 4 µg/ml aconitine and 0.016 µg/ml mercury cyanide.

## Revendications

1. Composition pharmaceutique comprenant :
a) l'atropine ou un de ses sels ;
b) l'aconitine ; et
c) le cyanure de mercure ;
destinée à l'emploi dans un procédé de traitement d'une infection virale, l'infection virale étant due à des virus de l'herpès, à des rhinovirus, au RSV (virus respiratoire syncytal humain) et/ou à des entérovirus.

2. Composition pharmaceutique destinée à l'emploi dans un procédé pour le traitement d'une infection virale selon la revendication 1, l'infection virale étant due au HRV₁₄ (rhinovirus de type 14 humain), au HSV₁ (virus de l'herpès simplex de type 1), et/ou du CA₉ (virus Coxsackie de type 9 humain).

3. Composition pharmaceutique destinée à l'emploi dans un procédé pour le traitement d'une infection virale selon une ou plusieurs des revendications précédentes, la composition se présentant sous la forme d'une solution aqueuse éthanolique et contenant environ 5 µg/ml de sulfate d'atropine, environ 1 µg/ml d'aconitine et environ 0,004 µg/ml de cyanure de mercure.

4. Composition pharmaceutique destinée à l'emploi dans un procédé pour le traitement d'une infection virale selon la revendication 1 ou 2, la composition contenant entre 1 et 10 µg/ml, préférentiellement de 2 à 7 µg/ml, le préférentiellement de 3,13 à 6,25 µg/ml de sulfate d'atropine.

5. Composition pharmaceutique destinée à l'emploi dans un procédé pour le traitement d'une infection virale selon une ou plusieurs des revendications 1 à 2, la composition contenant entre 0,05 et 5 µg/ml, préférentiellement de 0,5 à 2 µg/ml, le préférentiellement de 0,16 à 1,56 µg/ml d'aconitine.

6. Composition pharmaceutique destinée à l'emploi dans un procédé pour le traitement d'une infection virale selon une ou plusieurs des revendications 1 À 2, la composition contenant entre 0,001 et 0,1 µg/ml, préférentiellement de 0,001 à 0,01 µg/ml, le préférentiellement de 0,003 à 0,006 µg/ml de cyanure de mercure.

7. Composition pharmaceutique destinée à l'emploi dans un procédé pour le traitement d'une infection virale selon une ou plusieurs des revendications précédentes, la composition étant administrée à une dose journalière d'environ 20 µg/ml de sulfate d'atropine, 4 µg/ml d'aconitine et 0,016 µg/ml de cyanure de mercure.
